# EUROPEAN PATENT SPECIFICATION

(11) **EP 2 091 558 B1**
(45) Date of publication and mention of the grant of the patent: **04.04.2018**
(21) Application number: 07840065.2
(22) Date of filing: 16.11.2007
(51) Int. Cl.: A61K 39/00

(54) **METHODS OF ENHANCING IMMUNE RESPONSE USING ELECTROPORATION-ASSISTED VACCINATION AND BOOSTING**
VERFAHREN ZUR VERSTÄRKUNG DER IMMUNANTWORT MIT ELEKTROPORATIONSGESTÜTZTER IMPFUNG UND BOOSTING
METHODES ACCROISSANT LA REPONSE IMMUNITAIRE PAR VACCINATION ASSISTEE PAR ELECTROPORATION, ET RENFORT

(30) Priority: 17.11.2006 US 859724 P
(43) Date of publication of application: 26.08.2009
(73) Proprietor: Genetronics, Inc., San Diego, CA 92121-1318 (US)
(72) Inventor: MATHIESEN, Iacob, San Diego, CA 92121-1318 (US); TJELLE, Elisabeth Torunn, San Diego, CA 92121-1318 (US); KJEKEN, Rune, San Diego, CA 92121-1318 (US); RABUSSAY, Dietmar, San Diego, CA 92121-1318 (US); LIN, Feng, San Diego, CA 92121-1318 (US)
(74) Representative: Walker, Ross Thomson
(86) International application number: PCT/US2007/024051
(87) International publication number: WO 2008/063555

(56) References cited:
- GILBERT S C ET AL: "Synergistic DNA-MVA prime-boost vaccination regimes for malaria and tuberculosis", VACCINE, ELSEVIER LTD, GB, vol. 24, no. 21, 22 May 2006 (2006-05-22), pages 4554-4561, XP028010747, ISSN: 0264-410X, DOI: 10.1016/J.VACCINE.2005.08.048 [retrieved on 2006-05-22]
- SANTOSUOSSO MICHAEL ET AL: "Intranasal boosting with an adenovirus-vectored vaccine markedly enhances protection by parenteral Mycobactetium bovis BCG immunization against pulmonary tuberculosis", INFECTION AND IMMUNITY, AMERICAN SOCIETY FOR MICROBIOLOGY, WASHINGTON, US, vol. 74, no. 8, 1 August 2006 (2006-08-01) , pages 4634-4643, XP009129410, ISSN: 0019-9567
- LI Z ET AL: "DNA electroporation prime and protein boost strategy enhances humoral immunity of tuberculosis DNA vaccines in mice and non-human primates", VACCINE, ELSEVIER LTD, GB, vol. 24, no. 21, 22 May 2006 (2006-05-22), pages 4565-4568, XP025151379, ISSN: 0264-410X, DOI: 10.1016/J.VACCINE.2005.08.021 [retrieved on 2006-05-22]
- ZHANG ET AL.: 'Enhancement of the effectiveness of electroporation-augmented cutaneous DNA vaccination by a particulate adjuvant' BIOCHEMISTRY 2004, pages 369 - 373, XP008108995
- BABIUK ET AL.: 'Electroporation improves the efficacy of DNA vaccines in large animals' VACCINE vol. 20, 2002, pages 3399 - 3408, XP004378535
- SUDARSHAN ET AL.: "Boosting effect ot purified chick embryo cell rabies vaccine using the iontradermal route in person previously immunized by the intramuscular route or vice versa", THE NATIONAL MEDICAL JOURNAL OF INDIA, vol. 19, July 2006 (2006-07), pages 192-194, XP009170714,
- S. Buchan ET AL: "Electroporation as a "Prime/Boost" Strategy for Naked DNA Vaccination against a Tumor Antigen", THE JOURNAL OF IMMUNOLOGY, vol. 174, no. 10, 5 May 2005 (2005-05-05), pages 6292-6298, XP055304453, US ISSN: 0022-1767, DOI: 10.4049/jimmunol.174.10.6292

## Description

### FIELD OF THE INVENTION

This invention relates to the use of vaccines. More particularly, this disclosure relates to inducing and enhancing an immune response in a mammal to an antigenic agent by providing to said mammal a primary administration of said antigenic agent in a first immune responsive tissue and thereafter providing to said mammal at least one boost administration in a second body tissue wherein said prime and boost administrations are assisted by electroporation.

### BACKGROUND OF THE INVENTION

The following description includes information that may be useful in understanding the present invention. It is not an admission that any such information is prior art, or relevant, to the presently claimed inventions, or that any publication specifically or implicitly referenced is prior art.

In the vaccination arts, administration of a vaccine is typically by injection with a syringe and needle into either subcutaneous or intramuscular (IM) tissue. In conventional practice, the initial administration of the vaccine is followed by one or more boosting injections that are administered in the same manner as the first injection, only at a later time or times, typically, 2 to 6 weeks from initial administration. In some cases, a second boost administration is delivered at a second interval, which is often longer than the first interval (i.e., the time between the initial, or "priming", administration of the vaccine and the first boost vaccination). During such intervals, the immune system typically responds, for example, by raising antibodies to the antigenic component(s) of the vaccine (a so-called "humoral" immune) and/or by eliciting a cellular immune response involving cytotoxic T lymphocytes. In the context of antibody production, the level of the antibody titer generally reaches a maximum titer about 4 to 8 weeks post boosting.

With many diseases, boosting the immune system is necessary to keep the body in a state of readiness for fighting infection or the development of certain tumors. Buchan et al., 2005, Journal of Immunology, Vol. 174, pp. 6292-6298 discloses a vaccination protocol against tumors using DNA applied to muscle tissues. However, boosting in a single tissue type does not always provide an optimal immune response. Many factors influence the quality of an immune response, e.g., for example, some antigens do not induce a strong immune response, while other antigens may elicit induction of strong immune responses and inflammatory or sometimes regulatory immune responses. The variability of the immune system response to various antigenic agents is a major concern in vaccine development. With the advent of bioterrorism and the possibility of highly virulent strains of pathogens used as biological weapons, as well as the possible dangers posed by the evolution and potential rapid spread of mammalian and avian pathogens (e.g., HIV, SARS, H5N1, etc.) among and across species, there is a need in the art to provide for a methodology of vaccinating populations in a manner that can provide for the rapid induction of robust, broad immune responses in a consistent manner in large populations.

The present disclosure advances the vaccination arts in just such a manner by providing methods for enhancing the immune response to target antigens comprising the electroporation-assisted administration of priming and boosting compositions in preselected tissues.

### SUMMARY OF THE INVENTION

The present invention is as set out in the claims.

In one aspect, the present disclosure comprises the administration of vaccine compositions into skin and/or muscle tissues. In some embodiments of this aspect, the disclosure comprises dosing regimens for the administration of a vaccine composition wherein the initial bolus is administered in one tissue type (for example, muscle, skin, subcutaneous space, mucosa, intranasally, or inhaled in the lung) and the boosting administration(s) being delivered in a different, or second, tissue type. In a particularly preferred aspect of the present disclosure, the initial priming bolus is administrated to the skin and the boost bolus is administered in muscle tissue. In an alternate aspect of the present disclosure, the initial bolus can be administered into muscle and one or more boosting administrations can be delivered in skin tissue.

In another aspect the disclosure concerns the use of electroporation (EP) in the delivery of a vaccine composition administered by two or more dosings each separated by more than one day. Electroporation can be provided by any suitable electroporation device that is appropriate for delivering substances into cells within a tissue of a particular type. For example, for administration of a substance to skin tissue cells, an electroporation device can comprise any device having the capability to electroporate transdermally or transmucosally using non-invasive electrodes, or alternatively a device having needle-like electrodes that can electroporate tissue with electrodes inserted directly into the tissue to be electroporated, such as skin (either intradermally or subdermally), mucosa, or muscle.

Yet another aspect of the disclosure concerns electroporation-assisted methods of administering to a patient a vaccine so as to cause an enhanced immune response as compared to the immune response that would be expected to be observed without electroporation of the same quantity and quality of immunogen or that would be expected to be observed following administration in one tissue type alone.

In still a further aspect, the disclosure concerns the electroporation-assisted administration of a vaccine consisting of an antigen in the form of an attenuated or inactivated bacteria or virus; a protein, polypeptide, or peptide; or alternatively, administration of a nucleic acid (or multiple nucleic acid species) encoding one or more antigens, and particularly a nucleic acid capable of directing the expression of the encoded antigen(s) after administration to the body tissues and uptake by cells within such tissues.

In still another aspect the disclosure provides for immune responses that are faster, stronger, and inclusive of capability to illicit both humoral and/or cellular (i.e., T cell) immune responses.

These and other aspects and embodiments will become apparent by reference to the following drawings, detailed description, and appended claims.

### BRIEF DESCRIPTION OF THE DRAWINGS

Figure **1** is a graph showing resulting rabbit anti-human IgG antibody titers following injection and EP with DNA plasmid encoding human IgG in rabbits in selected tissues. Error bars represent the standard error of the mean.
Figures **2A** and **B** are graphs showing results for antibody production to Hepatitis B surface antigen in different cohorts of Balb/c mice. As depicted, initial inoculation in skin with a follow-up boost 6 days later in muscle combined with electroporation provides for enhanced titer level.
Figures **3A** and **B** are graphs showing results for antibody production to Hepatitis B surface antigen of different cohorts of Balb/c mice. As depicted, the results confirm those obtained in Figures **2A** and **B****.** Specifically, boosting in muscle in conjunction with electroporation even at 20 days after the initial inoculation provides for enhanced antibody titer.

### DETAILED DESCRIPTION OF THE INVENTION

The disclosure generally concerns methods for the electroporation-assisted delivery of vaccines to pathogens, infectious agents, and other disease states (e.g., cancer), which vaccine compositions are administered over time in two or more dosings. That said, however, the instant methods are based on the inventive recognition that an initial bolus delivery into a first tissue, for example, skin tissue, followed by boosting into a second, different tissue, for example, muscle tissue, while employing electroporation to directly deliver said vaccine compositions into the cells of at least one, and preferably each of, said tissues, provides for an enhanced immune response in a mammal. Indeed, in some preferred aspects of the present disclosure, the initial, or priming, administration comprises the elctroporation-assisted delivery of the antigenic agent(s) for treatment of the particular disease or disorder to skin tissue while one or more subsequent "boosting" administrations are delivered to muscle tissue that has been contemporaneously electroporated (i.e., electroporated before, simultaneously, or after delivery of the antigen(s) (or antigen-encoding nucleic acid(s) so as to enhances uptake of the antigen(s) (or nucleic acid(s)). In other preferred aspects of the present disclosure, priming is selectively performed by contemporaneous EP and administration of the initial bolus to muscle with subsequent boosting in skin tissues.

In the context of EP-assisted vaccine delivery, vaccines can be delivered to tissues using electroporation devices comprising various types of electrodes. For example, with regard to electroporation of a skin tissue, e.g., the stratum corneum, dermal, and subdermal tissues, an electroporation device can employ noninvasive electrodes such as meander electrodes and ring electrodes as described in U.S. patent nos. 6,009,345, 5,968,006, and 6,972,013. Additionally, noninvasive point electrodes, such as described in PCT application WO02/072781 and caliper electrodes as described in U.S. patent no. 5,439,440, can also be employed. In a related preferred aspect of the present disclosure, the vaccine can be delivered either through a topical application to the skin surface such that an electroporative pulse or pulse train is employed to carry the antigen through the surface of the stratum corneum and into deeper skin tissues by "transdermal electroporation", while in other preferred aspects of the present disclosure the vaccine is administered into the skin tissues (e.g., dermally or subdermally) using a needle and syringe, a needle-free jet injection device such as the Biojector, a skin patch delivery system, or any other suitable approach, in conjunction with contemporaneously providing to the skin tissue an electroporative pulse or pulse train using any suitable electrode configuration, for example, meander, ring, or point electrodes.

Vaccination in the skin tissues can also be carried out using invasive electrodes, such as needle-like electrodes, microelectrodes, or electrodes formed by conductive fluid jets ejected from jet injection devices. In some such aspects of the present disclosure, needle-like electrodes can also comprise a hollow needle that can be used to deliver the antigenic substance into the body tissue. With respect to skin administration, a vaccine composition can be, for example, injected into the skin tissue subdermally, such as by a separate hypodermic needle, jet injector, or through the electrodes themselves, followed by pulsing the electrodes with one or more electroporative pulses.

Regarding administration of vaccine compositions to muscle tissue, typically needle type electrodes, optionally capable of delivering vaccine, are pulsed contemporaneously with (often following) delivery of the vaccine to the muscle by needle and syringe or jet injection.

Needle electrodes used in the methods of the disclosure can comprise any suitable needle electrode adapted for the intended purpose, including those described in the above-stated patents or as described in any of U.S. patent nos. 5,273,525, 6,110,161, 6,261,281, 6,958,060, PCT application WO01/85202, and WO2007/095140.

As will be appreciated, the present invention provides for enhanced immune response due to the initial vaccination into a first tissue (e.g., a skin tissue) followed by boosting one or more times in a second tissue (e.g., a muscle tissue). Indeed, in embodiments that involve a plurality of boost administrations, second or subsequent boosts may be administered to still other tissue types than that to which the first boost dosage was administered. Without wishing to be bound to a particular theory, it is believed that an enhanced immune response is elicited, at least in part, by methods that involve priming in skin tissue and boosting in muscle because vaccination into skin provides a relatively higher concentration of dendritic cells capable of influencing the immune processing of the delivered antigenic agent(s) while boosting in muscle provides for relatively long term presence of the antigen(s) in the muscle compartment such that the antigen is processed in the cell providing a cellular response. Accordingly, delivery of one or more nucleic acid species encoding one or more peptide or polypeptide antigen species enhances long term expression of the peptide or polypeptide antigen(s), thereby allowing for relatively long term exposure of the expressed antigen(s) to the cellular and humoral arms of the immune system.

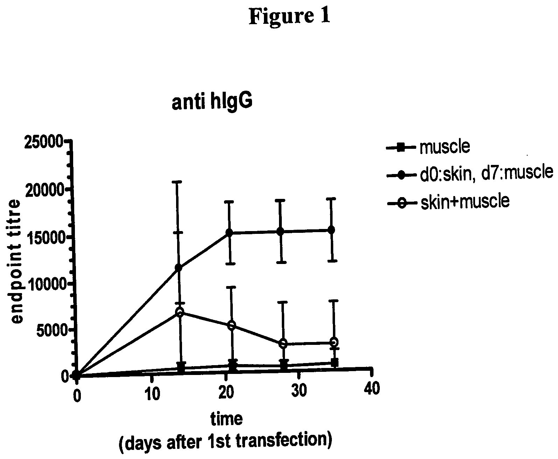

In yet another aspect of the present disclosure, the method of administration includes spacing the timing of the boost inoculum from the prime by an appropriate number of days. Of course, the particular intervals between priming and boosting (and between subsequent boosts of two or more boost administrations are contemplated or desired) can readily be ascertained, and will depend on such factors as the desired immune response to be elicited, antigen(s) being delivered, the tissue types into which prime and boost compositions were delivered, the type of compositions delivered (e.g., a peptide-containing composition in each instance, an antigen-encoding nucleic acid in each instance, a peptide in one instance (e.g., for priming) and a nucleic acid encoding the peptide in another (e.g., for boosting), whether or not EP is used in conjunction with priming, boosting, or priming and boosting, the age and condition of the patient to whom the compositions are to be administered, etc. As shown in the accompanying Figure 1, administration to both skin and muscle simultaneously is less optimal than spacing the timing between priming and boosting a sufficient period to allow the immune system to react to the prime inoculation in the skin. In a preferred aspect of the present disclosure, the administration of the vaccine prime bolus is spaced in time from the boost bolus by between one day and about 4-7 weeks. In a particularly preferred aspect of the present disclosure, the time interval between prime and boost boluses can be between one day and 7, 14, 20, 30, 35, 40, 45, 50 and 54 days. In particularly preferred aspects of the present disclosure, the interval can be 2 days, 6 days, or 20 days. When multiple boost dosages are administered, the interval between subsequent boosts can be the same or different. Some preferred between-boost intervals include one week, 2-4 weeks, 1-3 months, 4, 5, 6, 8, and 10 months, and 1, 2, 3, 4, 5, 6, 7, 8, 9, 10, 12, 15, and 20 years.

As already described, the vaccines of the invention can comprise, among other things, gene-based vaccines that encode one or more antigenic peptides, proteins, or polypeptides the expression of which is/are regulated or controlled by a promoter, preferably an inducible or tissue-specific promoter. In some preferred such aspect of the present disclosure, boluses are delivered over pre-determined intervals to both skin and muscle tissues for prime and boost, respectively. However, the disclosure also envisions contemporaneously administering both the prime and boost inoculations, with the administration of the priming bolus preferably being into skin and the boost inoculation being delivered into muscle. In a particularly preferred aspect of this sort, the vaccine composition delivered to the muscle comprises a nucleic acid that encodes the antigen under the regulation of an inducible promoter that can be induced upon exposure to or operable association with an inducing agent. Thus, at a selected time, such as, for example, between 1 to 14 days after a contemporaneous prime and boost, expression of the gene encoded by the nucleic acid delivered to the muscle tissue can be induced to express the antigen of interest and induce or enhance immune responsiveness to the particular antigen. In still other alternate aspects, the vaccine composition used for priming can comprise an expressible nucleic acid encoding an antigenic peptide while the boost composition can comprise a peptide or polypeptide comprising the antigen itself. That is, in such an aspect one or more vaccine compositions (be it a composition used for priming or boosting) can comprise a different form of the same antigen; for example, one of the compositions may include a single antigenic peptide, protein, or polypeptide species while another of the compositions may comprise a nucleic acid capable of directing the expression of the same antigenic peptide, protein, or polypeptide species. Further still, the prime and/or boost compositions can comprise any suitable gene delivery vehicle (e.g., a DNA or RNA virus, a viral genome, a "naked" DNA vector, a plasmid or other genetic element not intended to be integrated into the genome of the cell into which it is introduced, a vector or portion thereof intended for integration (by homologous recombination, random insertion, or otherwise), peptide, protein, or polypeptide species, and/or organic molecule cocktails designed for any particular indication where an immune response is desired.

Turning now to particular representative, non-limiting examples of the invention, experiments that involve administration of vaccine compositions to skin tissue followed by boosting in muscle (using electroporation in both instances) are described that provide for enhanced immune responses.

### Example 1

Three cohorts of white New Zealand rabbits (n= 4) were vaccinated with DNA encoding whole human IgG protein. The vaccinations were administered as follows for cohorts 1-3:
1) day 0: muscle injection only
2) day 0: skin inoculation; day 7: muscle injection, each with electroporation
3) day 0: skin and muscle both vaccinated

Electroporation was carried out using an Elgen 1000 (Inovio AS, Oslo, Norway) device having twin injection/electrodes capable of injecting and electroporating in muscle tissue and the BTX ECM 820 having a caliper electrode (9 mm x 9 mm) for skin electroporation. For both the skin and muscle administrations, the electric field was applied after intradermal or intramuscular injection of the vaccine solution, respectively. For skin, vaccinations comprised using a 28G needle to inject into rabbit dorsal skin 30 µg/30 µl of IgG-encoding DNA solution. For muscle, inoculations comprised i.m. injection in the rabbit quadriceps using 200 µl DNA/needle =400 µl/injection site or 20 µg/400 µl hIgG.

### Electroporation conditions:

Muscle: 250 mA, 20 ms, 2 pulses, 100 ms interval (10Hz), 2-needle array, 21 G needles, 2 mm distance between two needle electrodes, 200 µl/needle, 1 cm total insertion depth, 0.7 cm injection depth
Skin: 100 V/mm, 10ms, 5 pulses, 1 s interval, 30 µl/site.

As shown in Figure **1****,** antibody titer elicited against human IgG in rabbit was surprisingly and substantially higher when priming and boosting was carried out in skin and muscle, respectively, as opposed to vaccination carried out in muscle alone, or skin and muscle only as a primary inoculation. Thus, this experiment shows that spaced-in-time administration of antigenic agents to different tissues can enhance an immune response to such antigenic agent. Such methods provide for both faster and higher presence of circulating antibodies against said agent.

### Example 2

In this example, the surprising advantage of priming in skin and boosting in a secondary tissue such as muscle with the added advantage of electroporation is shown using a (BALB/c) mouse immune model wherein antigen-specific anti-IgG1 and anti-IgG2a antibody titers, as measured by ELISA, were studied. Use of both anti-IgG1 and anti-IgG2a antibodies here provides confirmation that an immunization regimen in accordance with the invention enhances responses associated with inflammatory cell-mediated immunity (also referred to as a TH1 response) as well as responses associated with regulatory humoral immunity (also referred to as a TH2 response).

In a first experiment, five cohorts of Balb/c mice (n=7) were vaccinated with 30µg/30µl of plasmid DNA (pDNA) encoding hepatitis B surface antigen (g-Wiz-HBsAg) (Aldevron LLC, Fargo, ND, USA) in either skin (dermal) or muscle tissue of the mouse quadriceps followed by electroporation. Electroporation was applied using an Elgen 1000 device (Inovio Biomedical Corp, San Diego) in muscle and using BTX ECM 820 in skin.

For muscle treatment electroporation was carried out using 50V and current limit at 250mA, 5 pulses, 20ms pulse length, 100 ms interval. For skin (dermal) tissue, electroporation was carried out using caliper electrodes at parameters: 3 pulses, 10ms pulse length, 150 V/mm, 1 s interval between the pulses.
Cohort 1 received the priming inoculation on day 0 in skin (intradermal) tissue (SO).
Cohort 2 received the priming inoculation on day 0 in muscle (M0).
Cohort 3 received a priming inoculation on day 0 in both skin (intradermal) and muscle (S0M0).
Cohort 4 received the priming inoculation on day 0 in skin (intradermal) and then was boosted at day 2 in muscle (S0M2).
Cohort 5 received the priming inoculations on day 0 in skin (intradermal) and then were boosted at day 6 in muscle (S0M6).

Serum anti-HBs specific immune responses were measured by ELISA. (A) Geometric mean anti-HBs IgG1 antibody endpoint titers (± standard error) were determined at different time points (day 14, 21, 28 and 42 after first immunization). Statistically significant differences at day 42 were noted, *p<0.05. (B) Geometric mean anti-HBs IgG2a endpoint titers (± standard error) were determined at different time points. Statistically significant differences at day 42 were also noted, *p<0.05.

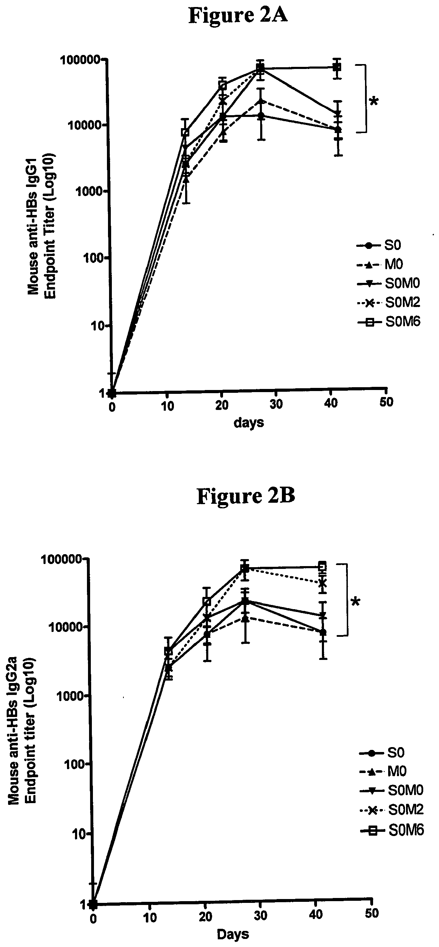

Results of the above protocol are shown in Figures **2A** and **B****.** As clearly indicated, this vaccination regimen provided an enhanced titer against a disease target antigen, e.g., a hepatitis B antigen. The treatment regimen enhanced both humoral as well as cellular responses. For example, Figure **2A** shows anti-HBs IgG1 antibody titers (humoral), and Figure **2B** shows immune response of anti-HBs IgG2a (cellular). Surprisingly, same time inoculations in two different tissues or, alternatively, only in one tissue type or another, e.g., skin and muscle, or skin or muscle, resulted in a lower titer than when the inoculation regimen comprised priming in skin and later boosting in muscle using electroporation. Further surprisingly, the enhanced effect was observed with as little as two days separating the prime and boost inoculations.

Further data shows that boosting in a secondary tissue, such a muscle, can be delayed from the first inoculation, here, by as much as 20 days, and still exhibit enhanced immune effect for an immunization regimen comprising priming in skin (dermal) tissue and boosting in muscle tissue, both by electroporation. Specifically, five cohorts of Balb/c mice (n=7 each cohort) were inoculated with 30µg/30µl of plasmid g-Wiz-HBsAg (Aldevron LLC, Fargo, ND) in either skin (dermal) or muscle tissue of the mouse quadriceps followed by electroporation. For muscle treatment, electroporation was carried out using an Elgen 1000 twin injector (Inovio Biomedical Corp, San Diego) with pulsing parameters set at 50V and current limit at 250mA, 5 pulses, 20ms pulse length, 100 ms interval between pulses. For skin/dermal tissue treatment, electroporation was carried out using caliper electrodes and pulse parameters comprising 3 pulses, 10ms pulse length, 150 V/mm, 1 s interval.
Cohort 1 received inoculation on day 0 in skin (intradermal) tissue (SO).
Cohort 2 received inoculation on day 0 in skin (intradermal) and then boosted again in skin at day 20 (S0S20).
Cohort 3 received inoculation on day 0 in muscle and then boosted at day 20 again in muscle (M0M20).
Cohort 4 received inoculation on day 0 in skin (intradermal) and then boosted at day 20 in muscle (S0M20).
Cohort 5 received inoculation on day 0 in muscle and then boosted at day 2 in skin (intradermal) (M0S20).

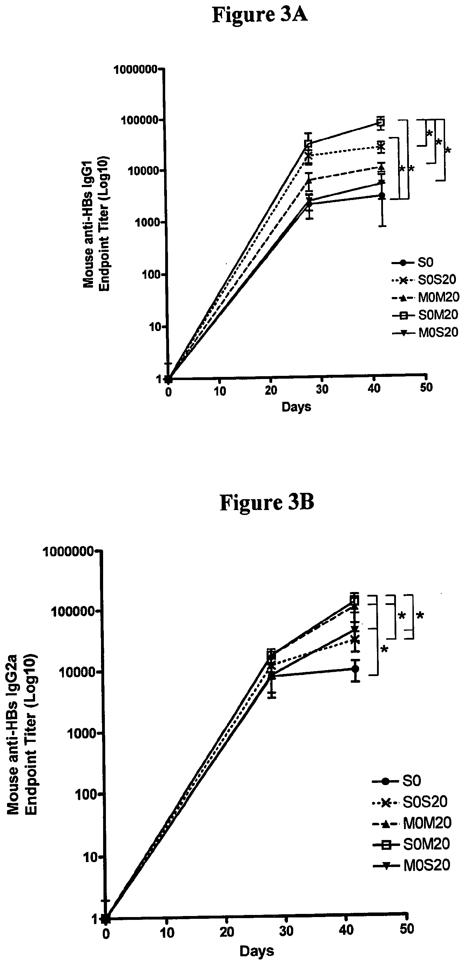

Results from this experiment appear in Figures **3A** and **B****.** Specifically, serum anti-HBs specific immune responses were measured by ELISA. In Figure **3A****,** geometric mean anti-HBs IgG1 antibody (humoral response) endpoint titers (± standard error) are presented at day 28 and day 42 after first vaccination. Statistically significant differences at day 42 are shown as *p<0.05. In Figure **3B****,** geometric mean anti-HBs IgG2a responses (cellular), endpoint titers ± standard error, are shown. Statistically significant differences (*p<0.05) at day 42 are noted. From these results it is clear that there is a distinct advantage of priming a patient with a vaccine in one tissue type and boosting in a second tissue type. In a particularly preferred aspect, there is realized an advantage in priming via electroporation in skin/dermal tissues and boosting in muscle via electroporation. Further, there is observed a benefit to boosting after a delay in time from boosting of at least 2 days, preferably 6 and/or 7 days, and alternately preferable after 7 days, such as at day 20, after the first inoculation.

All of the compositions and methods disclosed and claimed herein can be made and executed without undue experimentation in light of the present disclosure.

All patents, patent applications, and publications mentioned in the specification are indicative of the levels of those of ordinary skill in the art to which the invention pertains.

The invention illustratively described herein suitably may be practiced in the absence of any element(s) not specifically disclosed herein. Thus, for example, in each instance herein any of the terms "comprising", "consisting essentially of", and "consisting of" may be replaced with either of the other two terms. The terms and expressions which have been employed are used as terms of description and not of limitation, and there is no intention that use of such terms and expressions imply excluding any equivalents of the features shown and described in whole or in part thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

The invention concerns patentable processes and methods. A "patentable" process or method according to the invention, just as with any patentable machine, article of manufacture, composition of matter, means that the subject matter satisfies all statutory requirements for patentability at the time the analysis is performed. For example, with regard to novelty, non-obviousness, or the like, if later investigation reveals that one or more of the appended claims encompass one or more embodiments that would negate novelty, non-obviousness, *etc.,* the claim(s), being limited by definition to "patentable" embodiments, specifically exclude the unpatentable embodiment(s). Also, the claims appended hereto are to be interpreted both to provide the broadest reasonable scope, as well as to preserve their validity. Furthermore, if one or more of the statutory requirements for patentability are amended or if the standards change for assessing whether a particular statutory requirement for patentability is satisfied from the time this application is filed or issues as a patent to a time the validity of one or more of the appended claims is questioned, the claims are to be interpreted in a way that (1) preserves their validity and (2) provides the broadest reasonable interpretation under the circumstances.

## Claims

1. The use of a vaccine composition for the manufacture of a medicament for use in a method of enhancing an immune response in a mammal **characterised in that** the method comprises administering to said mammal a first bolus of the vaccine composition at a first time in a first tissue delivery site followed by administering to said mammal a boost bolus of said vaccine composition in a second tissue delivery site at a second time, wherein said boost bolus of said vaccine is administered to said mammal between 1 day and 20 days after the first bolus of the vaccine;
wherein the first tissue delivery site is skin tissue and the second tissue delivery site is muscle tissue, and
wherein said administration of said first and boost bolus comprises an electroporating electric pulse prior to, simultaneous with, or following administration of said first and boost bolus.

2. The use of claim 1 wherein said boost bolus of said vaccine is administered to said mammal between 2 days and 10 days after the first bolus of the vaccine.

3. The use of claim 1 or 2, wherein the first tissue delivery site is selected from the group consisting of skin, dermal and subdermal tissue and/or the second tissue delivery site is selected from the group consisting of muscle, striated muscle and smooth muscle.

4. The use of any one of the preceding claims wherein said mammal is a human.

5. The use of any one of claims 1 to 3 wherein said mammal is an animal.

6. The use of any one of the preceding claims wherein said vaccine composition comprises a polypeptide antigen.

7. The use of any one of the preceding claims wherein said vaccine composition comprises a nucleic acid encoding an antigen, said antigen capable of being expressed from said nucleic acid in said mammal.

8. A vaccine composition for use in a method of enhancing an immune response in a mammal **characterised in that** the method comprises administering to said mammal a first bolus of the vaccine composition at a first time in a first tissue delivery site followed by administering to said mammal a boost bolus of said vaccine composition in a second tissue delivery site at a second time, wherein said boost bolus of said vaccine is administered to said mammal between 1 day and 20 days after the first bolus of the vaccine;
wherein the first tissue delivery site is skin tissue and the second tissue delivery site is muscle tissue, and
wherein said administration of said first and boost bolus comprises an electroporating electric pulse prior to, simultaneous with, or following administration of said first and boost bolus.

9. The vaccine composition for the use of claim 8, wherein said boost bolus of said vaccine is administered to said mammal between 2 days and 10 days after the first bolus of the vaccine.

10. The vaccine composition for the use of claim 8 or 9, wherein the first tissue delivery site is selected from the group consisting of skin, dermal and subdermal tissue and/or the second tissue delivery site is selected from the group consisting of muscle, striated muscle and smooth
muscle.

11. The vaccine composition for the use of any one of claims 8 to 10, wherein said mammal is a human.

12. The vaccine composition for the use of any one of claims 8 to 10, wherein said mammal is an animal.

13. The vaccine composition for the use of any one of claims 8 to 12, wherein said vaccine composition comprises a polypeptide antigen.

14. The vaccine composition for the use of any one of claims 8 to 13, wherein said vaccine composition comprises a nucleic acid encoding an antigen, said antigen capable of being expressed from said nucleic acid in said mammal.

## Patentansprüche

1. Verwendung einer Impfstoffzusammensetzung für die Herstellung eines Medikaments zur Verwendung in einem Verfahren zur Verstärkung einer Immunantwort in einem Säugetier, **dadurch gekennzeichnet, dass** das Verfahren das Verabreichen, an besagtes Säugetier, eines ersten Bolus der Impfstoffzusammensetzung zu einem ersten Zeitpunkt in einer ersten Gewebeabgabestelle gefolgt vom Verabreichen, an besagtes Säugetier, eines Boost-Bolus der besagten Impfstoffzusammensetzung in einer zweiten Gewebeabgabestelle zu einem zweiten Zeitpunkt umfasst, worin besagter Boost-Bolus des besagten Impfstoffs zwischen 1 Tag und 20 Tagen nach dem ersten Bolus des Impfstoffs an besagtes Säugetier verabreicht wird;
worin die erste Gewebeabgabestelle Hautgewebe ist und die zweite Gewebeabgabestelle Muskelgewebe ist, und
worin besagte Verabreichung des besagten ersten und Boost-Bolus einen elektroporierenden elektrischen Impuls vor, gleichzeitig mit oder nach Verabreichung des besagten ersten und Boost-Bolus umfasst.

2. Verwendung nach Anspruch 1, worin besagter Boost-Bolus des besagten Impfstoffs zwischen 2 Tagen und 10 Tagen nach dem ersten Bolus des Impfstoffs an besagtes Säugetier verabreicht wird.

3. Verwendung nach Anspruch 1 oder 2, worin die erste Gewebeabgabestelle ausgewählt ist aus der Gruppe bestehend aus Haut, dermalem und subdermalem Gewebe und/oder die zweite Gewebeabgabestelle ausgewählt ist aus der Gruppe bestehend aus Muskulatur, quergestreifter Muskulatur und glatter Muskulatur.

4. Verwendung nach einem der vorhergehenden Ansprüche, worin besagtes Säugetier ein Mensch ist.

5. Verwendung nach einem der Ansprüche 1 bis 3, worin besagtes Säugetier ein Tier ist.

6. Verwendung nach einem der vorhergehenden Ansprüche, worin besagte Impfstoffzusammensetzung ein Polypeptidantigen umfasst.

7. Verwendung nach einem der vorhergehenden Ansprüche, worin besagte Impfstoffzusammensetzung eine Nukleinsäure, die für ein Antigen codiert, umfasst, wobei besagtes Antigen fähig ist, aus besagter Nukleinsäure in besagtem Säugetier exprimiert zu werden.

8. Impfstoffzusammensetzung zur Verwendung in einem Verfahren zur Verstärkung einer Immunantwort in einem Säugetier, **dadurch gekennzeichnet, dass** das Verfahren das Verabreichen, an besagtes Säugetier, eines ersten Bolus der Impfstoffzusammensetzung zu einem ersten Zeitpunkt in einer ersten Gewebeabgabestelle gefolgt vom Verabreichen, an besagtes Säugetier, eines Boost-Bolus der besagten Impfstoffzusammensetzung in einer zweiten Gewebeabgabestelle zu einem zweiten Zeitpunkt umfasst, worin besagter Boost-Bolus des besagten Impfstoffs zwischen 1 Tag und 20 Tagen nach dem ersten Bolus des Impfstoffs an besagtes Säugetier verabreicht wird;
worin die erste Gewebeabgabestelle Hautgewebe ist und die zweite Gewebeabgabestelle Muskelgewebe ist, und
worin besagte Verabreichung des besagten ersten und Boost-Bolus einen elektroporierenden elektrischen Impuls vor, gleichzeitig mit oder nach Verabreichung des besagten ersten und Boost-Bolus umfasst.

9. Impfstoffzusammensetzung zur Verwendung nach Anspruch 8, worin besagter Boost-Bolus des besagten Impfstoffs zwischen 2 Tagen und 10 Tagen nach dem ersten Bolus des Impfstoffs an besagtes Säugetier verabreicht wird.

10. Impfstoffzusammensetzung zur Verwendung nach Anspruch 8 oder 9, worin die erste Gewebeabgabestelle ausgewählt ist aus der Gruppe bestehend aus Haut, dermalem und subdermalem Gewebe und/oder die zweite Gewebeabgabestelle ausgewählt ist aus der Gruppe bestehend aus Muskulatur, quergestreifter Muskulatur und glatter Muskulatur.

11. Impfstoffzusammensetzung zur Verwendung nach einem der Ansprüche 8 bis 10, worin besagtes Säugetier ein Mensch ist.

12. Impfstoffzusammensetzung zur Verwendung nach einem der Ansprüche 8 bis 10, worin besagtes Säugetier ein Tier ist.

13. Impfstoffzusammensetzung zur Verwendung nach einem der Ansprüche 8 bis 12, worin besagte Impfstoffzusammensetzung ein Polypeptidantigen umfasst.

14. Impfstoffzusammensetzung zur Verwendung nach einem der Ansprüche 8 bis 13, worin besagte Impfstoffzusammensetzung eine Nukleinsäure, die für ein Antigen codiert, umfasst, wobei besagtes Antigen fähig ist, aus besagter Nukleinsäure in besagtem Säugetier exprimiert zu werden.

## Revendications

1. L'utilisation d'une composition de vaccin pour la fabrication d'un médicament pour une utilisation dans un procédé de renforcement d'une réponse immunitaire chez un mammifère **caractérisé en ce que** le procédé consiste à administrer audit mammifère un premier bolus de la composition de vaccin à un premier moment dans un premier site de distribution de tissu, puis à administrer audit mammifère un bolus de renfort de ladite composition de vaccin dans un deuxième site de distribution de tissu à un deuxième moment, où ledit bolus de renfort dudit vaccin est administré audit mammifère entre 1 jour et 20 jours après le premier bolus du vaccin;
où le premier site de distribution de tissu est un tissu cutané et le deuxième site de distribution de tissu est un tissu musculaire, et
où ladite administration dudit premier bolus de renfort comprend une impulsion électrique d'électroporation avant, simultanément à, ou suivant, l'administration dudit premier bolus de renfort.

2. L'utilisation selon la revendication 1 où ledit bolus de renfort dudit vaccin est administré audit mammifère entre 2 jours et 10 jours après le premier bolus du vaccin.

3. L'utilisation selon la revendication 1 ou 2, où le premier site de distribution de tissu est sélectionné dans le groupe constitué par le tissu cutané, dermique et sous-cutané et/ou le deuxième site de distribution de tissu est sélectionné dans le groupe constitué par le muscle, le muscle strié et le muscle lisse.

4. L'utilisation selon l'une quelconque des revendications précédentes où ledit mammifère est un humain.

5. L'utilisation selon l'une quelconque des revendications 1 à 3 où ledit mammifère est un animal.

6. L'utilisation selon l'une quelconque des revendications précédentes où ladite composition de vaccin comprend un antigène polypeptidique.

7. L'utilisation selon l'une quelconque des revendications précédentes où ladite composition de vaccin comprend un acide nucléique codant un antigène, ledit antigène pouvant être exprimé par ledit acide nucléique dans ledit mammifère.

8. Une composition de vaccin pour l'utilisation dans un procédé de renforcement d'une réponse immunitaire chez un mammifère **caractérisé en ce que** le procédé consiste à administrer audit mammifère un premier bolus de la composition de vaccin à un premier moment dans un premier site de distribution de tissu, puis à administrer audit mammifère un bolus de renfort de ladite composition de vaccin dans un deuxième site de distribution de tissu à un deuxième moment, où ledit bolus de renfort dudit vaccin est administré audit mammifère entre 1 jour et 20 jours après le premier bolus du vaccin;
où le premier site de distribution de tissu est un tissu cutané et le deuxième site de distribution de tissu est un tissu musculaire, et
où ladite administration dudit premier bolus de renfort comprend une impulsion électrique d'électroporation avant, simultanément à, ou suivant, l'administration dudit premier bolus de renfort.

9. La composition de vaccin pour l'utilisation selon la revendication 8, où ledit bolus de renfort dudit vaccin est administré audit mammifère entre 2 jours et 10 jours après le premier bolus du vaccin.

10. La composition de vaccin pour l'utilisation selon la revendication 8 ou 9, où le premier site de distribution de tissu est sélectionné dans le groupe constitué par le tissu cutané, dermique et sous-cutané et/ou le deuxième site de distribution de tissu est sélectionné dans le groupe constitué par le muscle, le muscle strié et le muscle lisse.

11. La composition de vaccin pour l'utilisation selon l'une quelconque des revendications 8 à 10, où ledit mammifère est un humain.

12. La composition de vaccin pour l'utilisation selon l'une quelconque des revendications 8 à 10, où ledit mammifère est un animal.

13. La composition de vaccin pour l'utilisation selon l'une quelconque des revendications 8 à 12, où ladite composition de vaccin comprend un antigène polypeptidique.

14. La composition de vaccin pour l'utilisation selon l'une quelconque des revendications 8 à 13, où ladite composition de vaccin comprend un acide nucléique codant un antigène, ledit antigène pouvant être exprimé par ledit acide nucléique dans ledit mammifère.
